# EUROPEAN PATENT SPECIFICATION

(11) **EP 2 036 509 B1**
(45) Date of publication and mention of the grant of the patent: **24.09.2014**
(21) Application number: 07767343.2
(22) Date of filing: 21.06.2007
(51) Int. Cl.: A61B 17/02

(54) **SPREADER FOR HIGH TIBIAL OSTEOTOMY**
SPREIZER FÜR HOHE TIBIAOSTEOTOMIE
ÉCARTEUR POUR L'OSTÉOTOMIE TIBIALE HAUTE

(30) Priority: 30.06.2006 JP 2006180893
(43) Date of publication of application: 18.03.2009
(73) Proprietor: Olympus Terumo Biomaterials Corp., Tokyo 163-0914 (JP)
(72) Inventor: OJIMA, Satoshi, Nishitokyo-shi, Tokyo 202-0002 (JP); SHIBATA, Koichi, Hino-shi, Tokyo 191-0041 (JP); TAKEUCHI, Ryohei, Yokohama-shi, Kanagawa 236-0038 (JP)
(74) Representative: von Hellfeld, Axel
(86) International application number: PCT/JP2007/062517
(87) International publication number: WO 2008/001674

(56) References cited:
- JP-A- 2001 505 456
- JP-A- 2004 524 098
- US-A1- 2005 273 114
- US-A1- 2005 273 114

## Description

### Technical Field

The present invention relates to a spreader for high tibial osteotomy to be inserted into a cut made in the deformed femur or tibia of a patient with knee osteoarthritis, and to open the cut so as to make a space that allows insertion of an implant.

### Background Art

High tibial osteotomy has been conventionally carried out to correct an angle of the deformed femur or tibia of a patient with knee osteoarthritis (for example, refer to Patent Document 1 and Patent Document 2). High tibial osteotomy is a method in which a wedge-shaped piece of bone is resected from the upper tibia that constitutes one part of the knee joint of a patient with knee osteoarthritis, and the resulting cut faces are joined together. On the other hand, there is another high tibial osteotomy method in which a cut is made in the femur or tibia constituting the knee joint using a bone saw, and the cut is opened at a correction angle.
Patent Document 1:
   Japanese Unexamined Patent Application, Publication No. 2002-65682.
Patent Document 2:
   Japanese Unexamined Patent Application, Publication No. 2004-298259.
Patent Document 3:
   US Patent Application Publication, Publication No. US 2005/0273114 A1 discloses a system and a method for performing an opening wedge osteotomy in a tibia 10 comprising a mechanical jack 90, 300 configured for placement into the bone cut in the tibia 10 so as to distract the tibia 10 at the bone cut. The system comprises an expandable wedge implant 805 comprising two opposing sides 810 which are coupled with an opening device such as the mechanical jack 90 or 300. Expandable wedge implant 805 comprises two opposing sides 810 whose surfaces frame the perimeter of the bony void and preferably create an opening 815 within the perimeter of sides 810. A base side 820 fits into the opening of the wedge osteotomy and is attachable to sides 810. Each one of the sides 810 is transversely split 825 along its length to form two opposing frame members 830, 835.

### Disclosure of Invention

In the case of the method in which a cut provided in the femur or tibia is opened, it is necessary to retain the cut in the opened state during insertion so that a bone implant or an artificial bone can be inserted into the space made by opening the cut.

However, if the cut is opened with use of a spreader, the spreader itself interferes with the insertion of the implant such as a bone implant or an artificial bone. Moreover, if the spreader is taken out from the cut during insertion, the cut is no longer retained in the opened state but is rather closed, which leads to a concern of difficulty regarding the insertion of the implant.

The present invention takes the above situation into consideration with an object of providing a spreader for high tibial osteotomy capable of facilitating insertion of an implant while retaining a cut in an opened state.

In order to achieve the above object, the present invention provides the following solutions.

The present invention provides a spreader for high tibial osteotomy to be inserted into a cut made in the deformed femur or tibia of a patient with knee osteoarthritis, and to open the cut so as to make a space that allows insertion of an implant, comprising: two pairs of swinging members which are respectively connected in a relatively swingable manner by hinge parts disposed at the distal end; and two opening/closing mechanisms which open/close these two pairs of swinging members respectively about the axes of the hinge parts, wherein the two pairs of swinging members are detachably assembled in the axial directions of the hinge parts.

The spreader for high tibial osteotomy according to the present invention is capable of: inserting the two pairs of swinging members in an assembled state from the hinge part side at the distal end into a cut made in the deformed femur or tibia of a patient with knee osteoarthritis; relatively swinging the swinging members in the opening direction about the axes of the hinge parts through operation of the opening/closing mechanisms, so as to open the cut by pressing cut faces of the cut with the swinging members. At this time, since the cut faces of the cut are pressed in wide areas by the two pairs of assembled swinging members, the contact pressure on the cut faces can be distributedly reduced, which enables to open the cut without damaging the cut faces.

Moreover, after the cut has been opened, either pair of the swinging members is swung in the closing direction about the axis of the hinge part through operation of one of the opening/closing mechanisms. By so doing, a space that allows insertion of an implant into the cut can be secured by taking out the pair of the closed swinging members while retaining the cut in an opened state with the other pair of the swinging members. Then, after the implant has been inserted, the other pair of the swinging members is swung in the closing direction about the axis of the hinge part, and thereby the space that allows insertion of the implant is secured by taking out the closed swinging members while retaining the cut in the opened state with the inserted implant. Therefore, the implant can be readily inserted into the entire opened cut.

Preferably in the spreader for high tibial osteotomy according to the above aspect, the two pairs of swinging members are respectively formed in an approximately wedge-shape which becomes gradually thicker from the distal end side in the closed state.

By so doing, insertion from the tapered distal end side that is provided with the hinge parts into the cut can be facilitated. Accordingly, at the time of insertion of the spreader for high tibial osteotomy into the cut, excessive load against the cut faces of the cut can be avoided, and the cut faces can be retained in a healthy state.

Moreover, in the above aspect, the structure may be such that one pair of said two pairs of swinging members is provided with engaging parts which are to be engaged with inner sides in the opening/closing direction of the other pair of the swinging members when assembled with the other pair of the swinging members.

By so doing, when the pair of swinging members provided with the engaging parts is mutually opened through operation of the opening/closing mechanism provided on the concerned pair of swinging members, the engaging parts are engaged with the inner sides in the opening/closing direction of the other pair of swinging members so that a pressure is applied in the relatively opening direction. Accordingly, the two pairs of swinging members can be simultaneously opened through operation of the single opening/closing mechanism, and the cut-open operation can be facilitated.

Furthermore, in the above structure, the opening/closing mechanism which is not provided with the engaging parts, among said two opening/closing mechanisms, may be constituted by: a screw hole provided on one of the swinging members connected by the hinge part; and a push screw to be fastened into the screw hole so as to press against the other swinging member in the opening direction.

By so doing, the opening/closing mechanism comprising the screw hole and the push screw will not restrict an operation by an external force to open the swinging members provided with the concerned opening/closing mechanism. Accordingly, the two pairs of swinging members can be readily and simultaneously opened by an external force applied from the engaging parts through operation of the other opening/closing mechanism. Moreover, after the swinging members have been opened, by fastening the push screw until the distal end thereof is abutted against the other swinging member, the cut can be retained in the opened state even if the other swinging members are taken out.

The present invention demonstrates an effect of facilitating insertion of an implant while retaining the cut in an opened state.

### Brief Description of Drawings

[FIG. 1] FIG. 1 is a longitudinal cross-sectional view of a spreader for high tibial osteotomy according to one embodiment of the present invention, which is taken along a section passing through a pair of swinging members.
[FIG. 2] FIG. 2 is a plan view showing the spreader for high tibial osteotomy of FIG. 1.
[FIG. 3] FIG. 3 is a plan view showing a state where two pairs of swinging members of the spreader for high tibial osteotomy of FIG. 1 are disassembled.
[FIG. 4] FIG. 4 is a longitudinal cross-sectional view of the spreader for high tibial osteotomy of FIG. 1, which is partially taken along a section passing through the other pair of swinging members.
[FIG. 5] FIG. 5 is a longitudinal cross-sectional view showing a state where the two pairs of swinging members of the spreader for high tibial osteotomy of FIG. 1 are swung in the opening direction.
[FIG. 6] FIG. 6 is a longitudinal cross-sectional view showing a state where two opening/closing mechanisms of the spreader for high tibial osteotomy of FIG. 1 are operated.

### Explanation of Reference Signs:

1: spreader for high tibial osteotomy
2a and 2b: first pair of swinging members
3a and 3b: second pair of swinging members
4: first opening/closing mechanism
5: second opening/closing mechanism
6 and 7: hinge parts
9: projection (engaging part)
11: screw hole
12: push screw

### Best Mode for Carrying Out the Invention

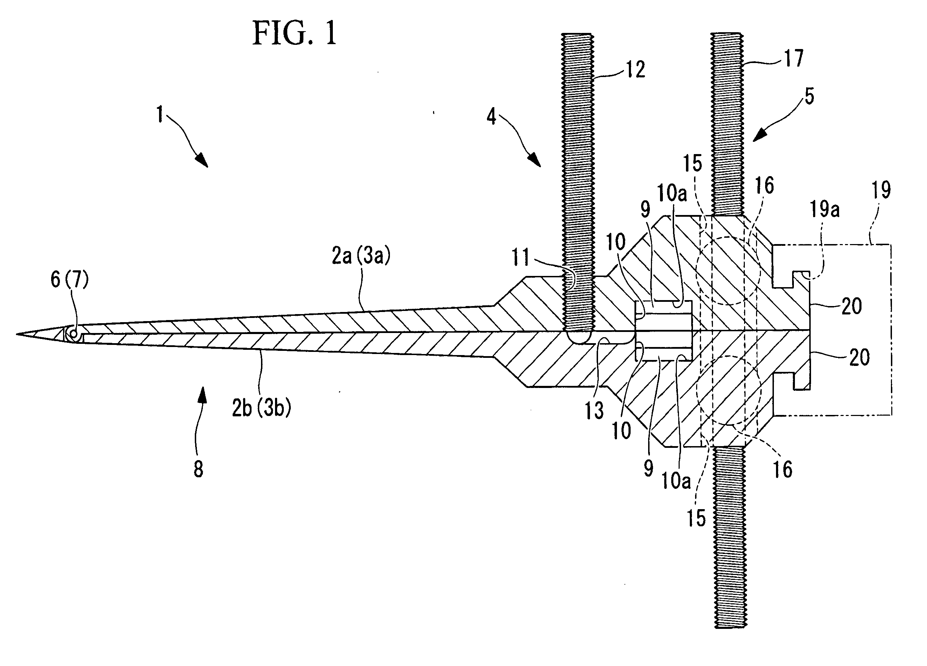

Hereunder is a description of a spreader 1 for high tibial osteotomy according to one embodiment of the present invention, with reference to FIG. 1 to FIG. 6.

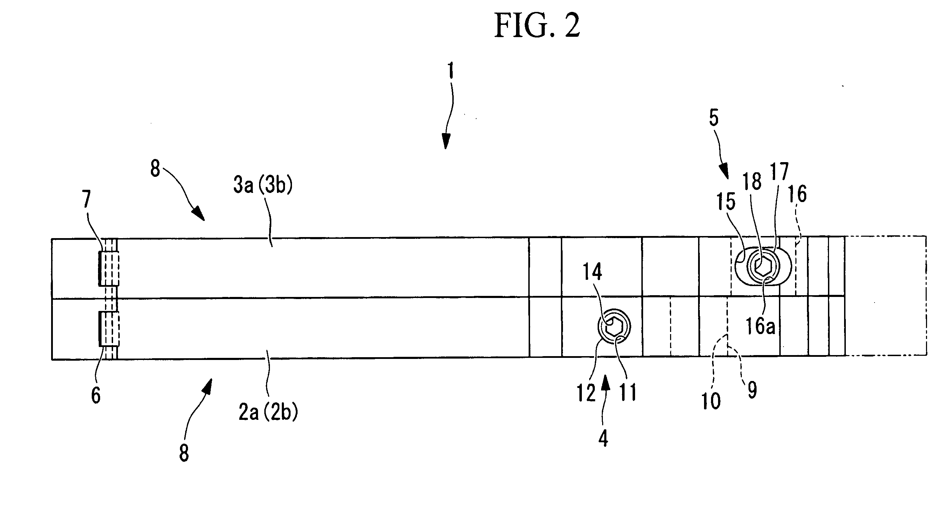

As shown in FIG. 1 and FIG. 2, the spreader 1 for high tibial osteotomy according to the present embodiment comprises first and second pairs of swinging members 2a/2b and 3a/3b, and first and second opening/closing mechanisms 4 and 5 which open/close the swinging members 2a/2b and 3a/3b.

These two pairs of swinging members 2a/2b and 3a/3b are respectively connected by hinge parts 6 and 7 disposed at the distal end, in a relatively swingable manner about the axes thereof. The respective pairs of the two swinging members 2a/2b and 3a/3b comprise wedge-shaped portions 8 in a wedge shape that is gradually tapered towards the distal end in a closed state where they are respectively in contact with each other. The wedge-shaped portion 8 is formed thin enough to be relatively readily inserted into a cut (not shown) made in the femur or tibia using a bone saw.

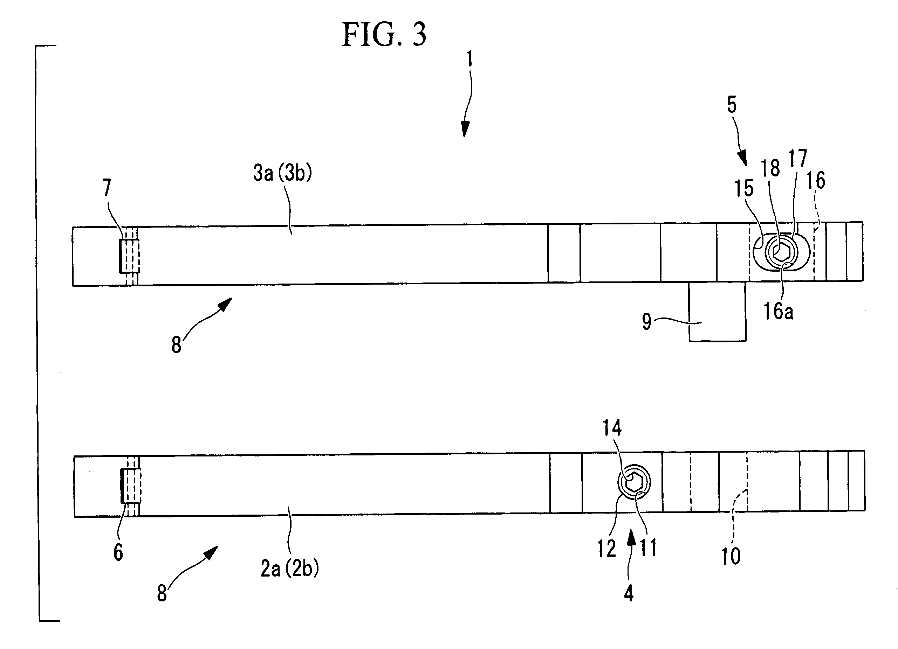

As shown in FIG. 3, the second pair of swinging members 3a/3b is respectively provided with a projection (engaging part) 9 which extends widthwisely outward from a widthwise side face thereof. Moreover, the first pair of swinging members 2a/2b is respectively provided with a concave portion 10 which accommodates the projection 9 when the two pairs of swinging members 2a/2b and 3a/3b are aligned in a close contact manner in the widthwise direction. As shown in FIG. 2, the two pairs of swinging members 2a/2b and 3a/3b are designed to be integrally assembled in a close contact manner in the widthwise direction by inserting the projections 9 into the concave portions 10. Furthermore, as shown in FIG. 3, the two pairs of swinging members 2a/2b and 3a/3b are designed to be readily detached in the widthwise direction by withdrawing the projections 9 from the concave portions 10.

Moreover, as shown in FIG. 1, when the projection 9 is inserted into the concave portion 10, the projection 9 is arranged to be abutted against an internal surface 10a in the opening/closing direction of the concave portion 10. As a result, by applying an external force so as to relatively open the second pair of swinging members 3a/3b which are provided with the projections 9, the external force is transmitted through the projections 9 to the internal surfaces 10a of the concave portions 10 engaged with the projections 9, so that the first pair of swinging members 2a/2b formed with the concave portions 10 is relatively opened.

As shown in FIG. 1, the first opening/closing mechanism 4 provided in the first pair of swinging members 2a/2b comprises a screw hole 11 formed in one swinging member 2a and a push screw 12 fastened thereto. The distal end of the push screw 12 is formed in a hemispherical shape so as to be stably contacted with the swinging member 2b regardless of changes in the relative angle between the two swinging members 2a/2b.

In the surface of the other swinging member 2b to be pressed by the distal end of the push screw 12 is formed a concave portion 13 which accommodates the distal end of the push screw 12, with an intention to stably perform the relative swinging operation of the two swinging members 2a/2b. As shown in FIG. 2, the other end of the push screw 12 is provided with a hexagonal hole 14 to be inserted with a tool (not shown) for facilitating the fastening operation.

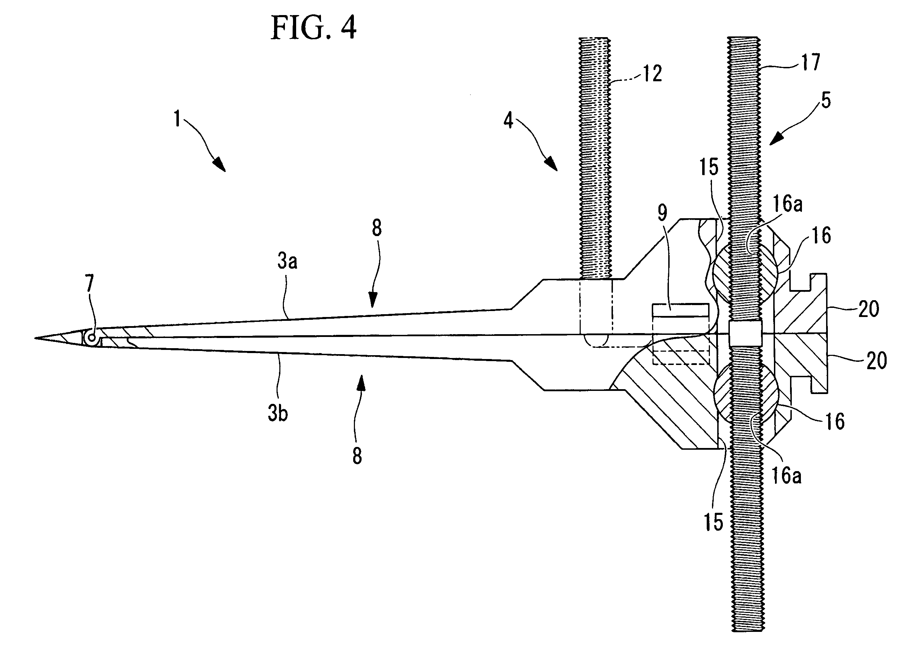

As shown in FIG. 4, the second opening/closing mechanism 5 provided on the second pair of swinging members 3a/3b comprises through holes 15 respectively formed through the swinging members 3a/3b along the opening/closing direction, spinning members 16 which are respectively arranged in a longitudinal midway position of each through hole 15 and are rotatably supported about axes parallel to the axis of the hinge part 7, and a bolt member 17 to be fastened piercingly to the screw holes 16a provided in the spinning members 16. The directions of male screws of the bolt member 17 are reversed at the longitudinal center. The respective male screws in a reverse screw relation of the bolt member 17 are fastened to the screw holes 16a of the spinning members 16 which are provided in the respective swinging members 3a/3b.

Moreover, one end of the bolt member 17 is provided with a hexagonal hole 18. Therefore, when a tool (not shown) is inserted into the hexagonal hole 18 and the bolt member 17 is rotated about the longitudinal axis, then the two spinning members 16 are moved in the opposite directions along the longitudinal axis of the bolt member 17, so as to change the relative angle between the two swinging members 3a/3b which are respectively attached with the spinning members 16.

In FIG. 1, the reference sign 19 indicated by the chain line denotes a striking block which has a dovetail groove 19a to be fitted to dovetail projections 20 respectively provided on the rear ends of the swinging members 2a, 2b, 3a, and 3b. By fitting this striking block 19 to the dovetail projections 20, the two pairs of swinging members 2a/2b and 3a/3b can be more firmly integrated. Also, by hitting this striking block 19 with a hammer (not shown) or the like from the rear side, the distal ends of the swinging members 2a, 2b, 3a, and 3b can be readily inserted into a cut made in the femur or tibia.

Hereunder is a description of the operation of thus constituted spreader 1 for high tibial osteotomy according to the present embodiment.

In order to implant an implant into a cut provided in the femur or tibia using the spreader 1 for high tibial osteotomy according to the present embodiment, firstly as shown in FIG. 2, the two pairs of swinging members 2a/2b and 3a/3b are integrally assembled in an adjacent manner by fitting the projections 9 into the concave portions 10. Then, the push screw 12 and the bolt member 17 of the two opening/closing mechanisms 4 and 5 are rotated respectively about their longitudinal axes, to thereby bring the two pairs of swinging members 2a/2b and 3a/3b in a closed state as shown in FIG. 1.

Furthermore, in this state, the striking block 19 is attached by fitting the dovetail groove 19a of the striking block 19 to the dovetail projections 20 formed at the rear ends of the two pairs of swinging members 2a, 2b, 3a, and 3b. By so doing, the two pairs of swinging members 2a/2b and 3a/3b are more firmly integrated.

In this state, since the wedge-shaped portions 8 at the distal ends of the swinging members 2a, 2b, 3a, and 3b are formed thin, the point of the wedge-shaped portions 8 is placed at the cut and inserted thereinto. In this case, by hitting the striking block 19 with a hammer or the like from the rear side, the wedge-shaped portions 8 are readily inserted into the cut by the impact force.

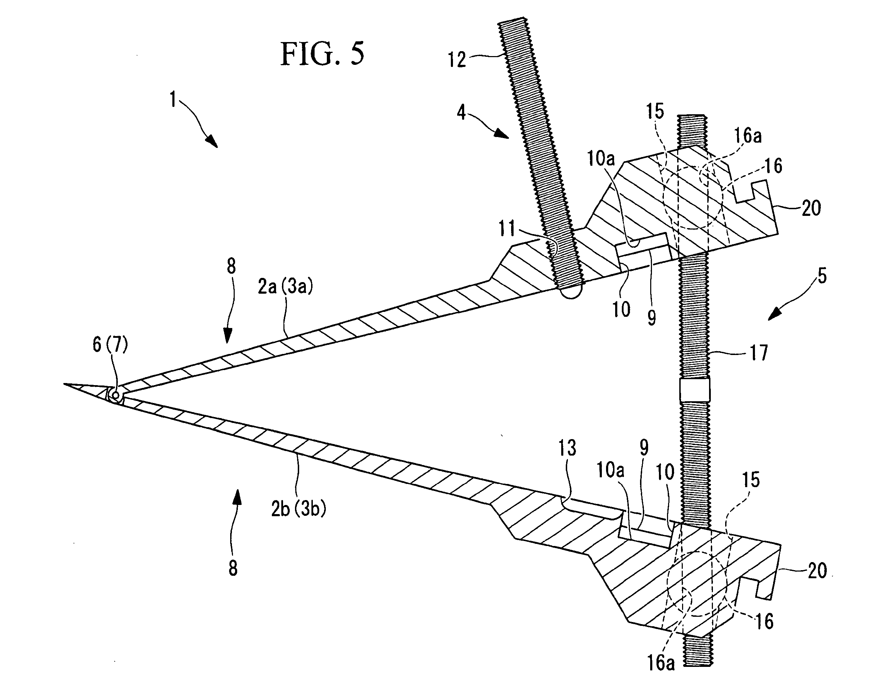

In a state where the wedge-shaped portions 8 are sufficiently inserted into the cut, the striking block 19 is taken out, and then the bolt member 17 constituting the second opening/closing mechanism 5 is rotated in one direction (for example, clockwise) about the longitudinal axis as shown in FIG. 5. The bolt member 17 is provided with male screws in the reverse screw relation, and the respective male screws are respectively fastened into two screw holes 16a in the spinning members 16. Therefore, by rotating the bolt member 17 in one direction about the longitudinal axis, the spinning members 16 can be moved towards relatively separating directions along the longitudinal axis of the bolt member 17.

By so doing, the relative angle between the second pair of swinging members 3a/3b attached with the spinning members 16, about the axis of the hinge part 7, is extended. At this time, the relative angles between the bolt member 17 and the second pair of swinging members 3a/3b are also changed. However, since the spinning members 16 are provided in the respective swinging members 3a and 3b in a rotatable manner about axes parallel to the axis of the hinge part 7, then the male screws of the bolt member 17 are retained in a fastened state to the two screw holes 16a in the spinning members 16 due to the rotations of respective spinning members 16.

In this case, according to the spreader 1 for high tibial osteotomy of the present embodiment, the projections 9 provided on the second pair of swinging members 3a/3b are fit in a contact manner in the internal surfaces 10a of the concave portions 10 which are provided in the first pair of swinging members 2a/2b. Therefore, by simply opening the second pair of swinging members 3a/3b through manipulation of the second opening/closing mechanism 5 of the second pair of swinging members 3a/3b, the first pair of swinging members 2a/2b can also be integrally opened via the projections 9 and the concave portions 10. Accordingly, the cut-open operation is facilitated.

In this manner, by simultaneously opening the two pairs of swinging members 2a/2b and 3a/3b in the assembled state, the cut faces of the cut is simultaneously pressed in wide areas by the two pairs of swinging members 2a/2b and 3a/3b. As a result, locally excessive pressure against the cut faces of the cut can be avoided, and the cut can be opened while keeping the femur or tibia in a healthy state without damaging it.

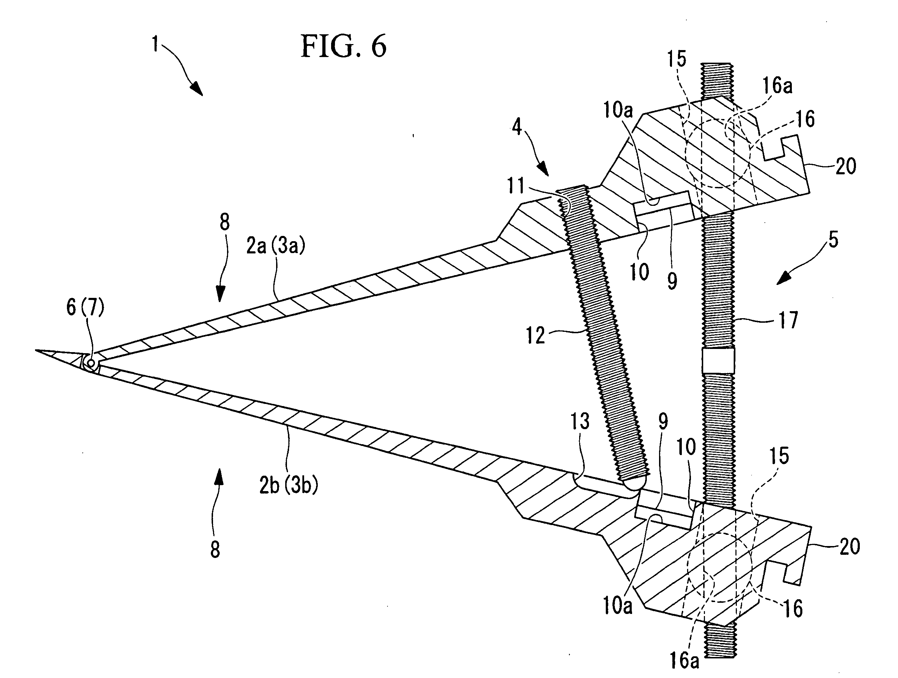

Next, in a state where the cut is opened until an enough space for insertion of an implant can be secured, the push screw 12 constituting the first opening/closing mechanism 4 is rotated about the longitudinal axis so as to bring the distal end of the push screw 12 into contact with the other swinging member 2b as shown in FIG. 6. By so doing, the first pair of swinging members 2a/2b provided with the first opening/closing mechanism 4 is also retained in an opened state by itself.

In this state, the bolt member 17 of the second opening/closing mechanism 5 is rotated in the opposite direction to the abovementioned direction. By so doing, the second pair of swinging members 3a/3b is displaced in the closing direction. At this time, the engagements between the concave portions 10 and the projections 9 in contact with the internal surfaces 10a thereof are released, so that only the second pair of swinging members 3a/3b is closed. The first pair of swinging members 2a/2b is retained in the opened state by the operation of the first opening/closing mechanism 4. Therefore, even if the second pair of swinging members 3a/3b is closed, the cut is sustained in the opened state by the first pair of swinging members 2a/2b.

Then, by taking out the second pair of closed swinging members 3a/3b from the inside of the cut, an enough space for insertion of an implant can be secured in a void space where the second pair of swinging members 3a/3b used to be disposed.

In this state, an implant such as an artificial bone or a bone implant in a shape matching with the space is inserted into the space.

Next, the push screw 12 of the first opening/closing mechanism 4 of the first pair of swinging members 2a/2b is rotated in the opposite direction to the abovementioned direction. By so doing, the first pair of swinging members 2a/2b is displaced in the closing direction. At this time, the cut is retained in the opened state by the implant inserted into the space. Then, by taking out the first pair of closed swinging members 2a/2b from the inside of the cut, an enough space for insertion of an implant can be secured in a void space where the first pair of swinging members 2a/2b used to be disposed.

In this state, an implant such as an artificial bone or a bone implant in a shape matching with the space is inserted into the space. Therefore, the implant can be readily inserted into the cut.

The spreader 1 for high tibial osteotomy according to the present embodiment has been described regarding the case where the spreader has two pairs of swinging members 2a/2b and 3a/3b; however, instead of this, the spreader may have three or more pairs of swinging members which can be assembled in a mutually detachable manner.

Moreover, the bolt member 17 with reverse screws has been employed as the second opening/closing mechanism 5; however, instead of this, a push screw similar to the push screw 12 of the first opening/closing mechanism 4 may also be employed.

## Claims

1. A spreader (1) for high tibial osteotomy to be inserted into a cut made in the deformed femur or tibia of a patient with knee osteoarthritis, and to open the cut so as to make a space that allows insertion of an implant, comprising:
two pairs of swinging members (2a, 2b; 3a, 3b) which are respectively connected in a relatively swingable manner by hinge parts (6, 7) disposed at the distal end; **characterised in that** the spreader further comprises
two opening/closing mechanisms (4, 5) which open/close the two pairs of swinging members (2a, 2b; 3a, 3b) respectively about the axes of the hinge parts (6, 7),
wherein said two pairs of swinging members (2a, 2b; 3a, 3b) are detachably assembled in the axial directions of the hinge parts (6, 7).

2. A spreader (1) for high tibial osteotomy according to claim 1, wherein said two pairs of swinging members (2a, 2b; 3a, 3b) are respectively formed in an approximately wedge-shape which becomes gradually thicker from the distal end side in the closed state.

3. A spreader (1) for high tibial osteotomy according to claim 1, wherein one pair of said two pairs of swinging members (2a, 2b; 3a, 3b) is provided with engaging parts (9) which are to be engaged with inner sides (10) in the opening/closing direction of the other pair of the swinging members (2a, 2b; 3a, 3b) when assembled with the other pair of the swinging members (2a, 2b; 3a, 3b).

4. A spreader (1) for high tibial osteotomy according to claim 3, wherein the opening/closing mechanism (4, 5) which is not provided with said engaging parts (9), among said two opening/closing mechanisms (4, 5), is constituted by: a screw hole (11) provided on one of the swinging members (2a, 2b; 3a, 3b) connected by the hinge part (6, 7); and a push screw (12) to be fastened into the screw hole (11) so as to press against the other swinging member (2a, 2b; 3a, 3b) in the opening direction.

## Patentansprüche

1. Spreizer (1) für eine hohe Tibia-Osteotomie, der dazu vorgesehen ist, in einen Schnitt, der in einer deformierten Femur oder Tibia eines Patienten mit einer Kniegelenksarthrose gemacht wurde, eingeführt zu werden und den Schnitt zu öffnen, um so einen Raum zu schaffen, der die Einführung eines Implantats erlaubt, und der umfasst:
zwei Paare von Schwingelementen (2a, 2b; 3a, 3b), die durch Scharnierteile (6, 7), die an dem distalen Ende angeordnet sind, jeweils in einer relativ schwingbaren Art und Weise verbunden sind;
**dadurch gekennzeichnet, dass** der Spreizer ferner umfasst
zwei Öffnungs-/Schließmechanismen (4, 5), die die zwei Paare von Schwingelementen (2a, 2b; 3a, 3b) jeweils um die Achsen der Scharnierteile (6, 7) öffnen/schließen,
wobei die zwei Paare von Schwingelementen (2a, 2b; 3a, 3b) in den axialen Richtungen der Scharnierteile (6, 7) lösbar montiert sind.

2. Spreizer (1) für eine hohe Tibia-Osteotomie gemäß Anspruch 1, wobei die zwei Paare von Schwingelementen (2a, 2b; 3a, 3b) jeweils annähernd in Form eines Keils ausgebildet sind, der von der distalen Endseite aus in dem geschlossenen Zustand allmählich dicker wird.

3. Spreizer (1) für eine hohe Tibia-Osteotomie gemäß Anspruch 1, wobei ein Paar der zwei Paare von Schwingelementen (2a, 2b; 3a, 3b) mit Eingriffselementen (9) versehen ist, die dazu vorgesehen sind, mit Innenseiten (10) in der Öffnungs-/Schließrichtung des anderen Paars von Schwingelementen (2a, 2b; 3a, 3b) in Eingriff gebracht zu werden, wenn es mit dem anderen Paar von Schwingelementen (2a, 2b; 3a, 3b) verbunden ist.

4. Spreizer (1) für eine hohe Tibia-Osteotomie gemäß Anspruch 3, wobei der Öffnungs-/Schließmechanismus (4, 5) der zwei Öffnungs-/Schließmechanismen (4, 5), der nicht mit den Eingriffselementen (9) ausgestattet ist, gebildet ist durch: ein Schraubenloch (11), das an einem der Schwingelemente (2a, 2b; 3a, 3b) vorgesehen ist, das durch das Scharnierteil (6, 7) verbunden ist; und eine Druckschraube (12), die dazu vorgesehen ist, so in dem Schraubenloch (11) befestigt zu werden, dass sie in der Öffnungsrichtung gegen das andere Schwingelement (2a, 2b; 3a, 3b) drückt.

## Revendications

1. Écarteur (1) pour ostéotomie tibiale haute destiné à être inséré dans une découpe réalisée dans le fémur ou le tibia déformé d'un patient avec une ostéoarthrite du genou, et pour ouvrir la découpe de façon à ménager un espace qui permet l'insertion d'un implant, comprenant :
deux paires d'éléments oscillants (2a, 2b ; 3a, 3b) qui sont respectivement connectés d'une manière pouvant osciller relativement par des parties (6, 7) de charnière disposées à l'extrémité distale ; **caractérisé en ce que** l'écarteur comprend en outre
deux mécanismes (4, 5) d'ouverture/fermeture qui ouvrent/ferment les deux paires d'éléments oscillants (2a, 2b ; 3a, 3b) respectivement autour des axes des parties (6, 7) de charnière,
dans lequel lesdites deux paires d'éléments oscillants (2a, 2b ; 3a, 3b) sont assemblées de façon détachable dans les sens axiaux des parties (6, 7) de charnière.

2. Écarteur (1) pour ostéotomie tibiale haute selon la revendication 1, dans lequel lesdites deux paires d'éléments oscillants (2a, 2b ; 3a, 3b) sont respectivement formées à une forme approximativement en coin qui devient graduellement plus épaisse à partir du côté d'extrémité distale à l'état fermé.

3. Écarteur (1) pour ostéotomie tibiale haute selon la revendication 1, dans lequel une paire desdites deux paires d'éléments oscillants (2a, 2b ; 3a, 3b) est prévue avec des parties (9) d'engagement qui sont destinées à être engagées avec des côtés intérieurs (10) dans le sens d'ouverture/fermeture de l'autre paire des éléments oscillants (2a, 2b ; 3a, 3b) lorsqu'elle est assemblée avec l'autre paire des éléments oscillants (2a, 2b ; 3a, 3b).

4. Écarteur (1) pour ostéotomie tibiale haute selon la revendication 3, dans lequel le mécanisme (4, 5) d'ouverture/fermeture qui n'est pas prévu avec lesdites parties (9) d'engagement, parmi lesdits deux mécanismes (4, 5) d'ouverture/fermeture est constitué par : un trou (11) pour vis prévu sur un des éléments oscillants (2a, 2b ; 3a, 3b) connecté par la partie (6, 7) de charnière ; et une vis-poussoir (12) destinée à être fixée dans le trou (11) pour vis de façon à presser contre l'autre élément oscillant (2a, 2b ; 3a, 3b) dans le sens d'ouverture.
